# EUROPEAN PATENT APPLICATION

(11) **EP 2 657 702 A1**
(43) Date of publication of application: **30.10.2013**
(21) Application number: 11850209.5
(22) Date of filing: 22.12.2011
(51) Int. Cl.: G01N 33/543, G01N 21/78, G01N 27/327, G01N 27/416, G01N 33/53, C12N 15/115

(54) **ANALYTICAL DEVICE AND ANALYTICAL METHOD**

(30) Priority: 24.12.2010 JP 2010287590
(71) Applicant: NEC Soft, Ltd., Tokyo 136-8627 (JP)
(72) Inventor: HORII Katsunori, Tokyo 136-8627 (JP); KATOU Shintarou, Tokyo 136-8627 (JP); AKITOMI Jou, Tokyo 136-8627 (JP); WAGA Iwao, Tokyo 136-8627 (JP)
(74) Representative: Betten & Resch
(86) International application number: PCT/JP2011/079856
(87) International publication number: WO 2012/086772

(57) **Abstract**

The present invention provides a technique capable of simply analyzing a target to be analyzed. An analytical device of the present invention includes a basal plate; a nucleic acid element; and a detection section of detecting a signal. The nucleic acid element and the detection section are arranged on the basal plate. The nucleic acid element includes a first nucleic acid molecule and a second nucleic acid molecule. The first nucleic acid molecule is a nucleic acid molecule capable of binding to a target. The second nucleic acid molecule is a nucleic acid molecule capable of binding to streptavidin. When the target does not bind to the first nucleic acid molecule, a binding capacity of the second nucleic acid molecule to the streptavidin is inactivated. When the target binds to the first nucleic acid molecule, a binding capacity of the second nucleic acid molecule to the streptavidin is activated. The detection section detects binding between the second nucleic acid molecule and the streptavidin. The target is bound to the first nucleic acid molecule, so that the streptavidin is bound to the second nucleic acid molecule. Thus, the target can be analyzed through detecting the binding between the second nucleic acid molecule and the streptavidin using the detection device.

## Description

### Technical Field

The present invention relates to an analytical device and an analytical method.

### Background Art

It has been necessary in various fields of clinical treatment, food, an environment, and the like to detect an intended target. Interactions with the target are generally utilized to detect the target. Among them, a method using an antibody which specifically binds to the target has been widely used (patent document 1). In this method, a labeled antibody labeled with an enzyme is used, for example. Specifically, first, a target in a sample and the labeled antibody are bound to each other. Then, the target is analyzed through detecting a chromogenic reaction of the enzyme in the labeled antibody using a chromogenic substrate for the enzyme.

However, the antibody is obtained by immunizing an animal, so that the following problems arise. That is, a highly toxic target is deadly for the immunized animal. Furthermore, a low molecular target is difficult to be recognized as an antigen in the immunized animal. Therefore, it is really difficult to obtain antibodies which specifically bind to these targets. Thus, a detectable target is limited.

Moreover, there is a problem in that a treatment process is complicated in the case of using the antibodies. Furthermore, it is necessary to easily analyze many samples in clinical treatment and the like, so that it is required to easily perform the detection method using a small device. However, it is difficult to downsize a device in the case of using the antibodies.

### Prior Art Documents

### Patent Document

Patent Document 1: JP 2009-133712 A

### Summary of Invention

### Problem to be Solved by the Invention

Hence, the present invention is intended to provide a novel technique capable of simply analyzing an objective target.

### Means for Solving Problem

The analytical device according to the present invention is an analytical device including: a basal plate; a nucleic acid element; and a detection section of detecting a signal, wherein the nucleic acid element and the detection section are arranged on the basal plate, the nucleic acid element includes a first nucleic acid molecule and a second nucleic acid molecule, the first nucleic acid molecule is a nucleic acid molecule capable of binding to a target, the second nucleic acid molecule is a nucleic acid molecule capable of binding to streptavidin, when the target does not bind to the first nucleic acid molecule, a binding capacity of the second nucleic acid molecule to the streptavidin is inactivated, when the target binds to the first nucleic acid molecule, a binding capacity of the second nucleic acid molecule to the streptavidin is activated, and the detection section detects binding between the second nucleic acid molecule and the streptavidin.

The analytical method for analyzing a target according to the present invention is an analytical method for analyzing a target using the analytical device according to the present invention, the analytical method including: an adding step of adding a sample and streptavidin to the analytical device; and a detecting step of detecting binding between the second nucleic acid molecule and the streptavidin in the detection section, whereby detecting a target.

### Effects of the Invention

According to the present invention, an object to be analyzed can be simply analyzed. An aptamer technology can be applied to the present invention, for example. Aptamers are obtained in a test tube, for example. Therefore, even aptamers capable of binding to a highly toxic compound and aptamers capable of binding to a low molecular compound can be obtained. Thus, according to the present invention, for example, problems with a method using an antigen-antibody reaction and the like can be solved. Further, according to the present invention, for example, a simple analysis by signal detection such as electrochemical detection or the like can be performed. Furthermore, according to the present invention, for example, since the nucleic acid element is used, it is possible to downsize the device and make the device into a chip. Thus, many samples can be analyzed easily. In the present invention, "analysis" encompasses quantitative analysis, semi-quantitative analysis, and qualitative analysis.

### Brief Description of Drawing

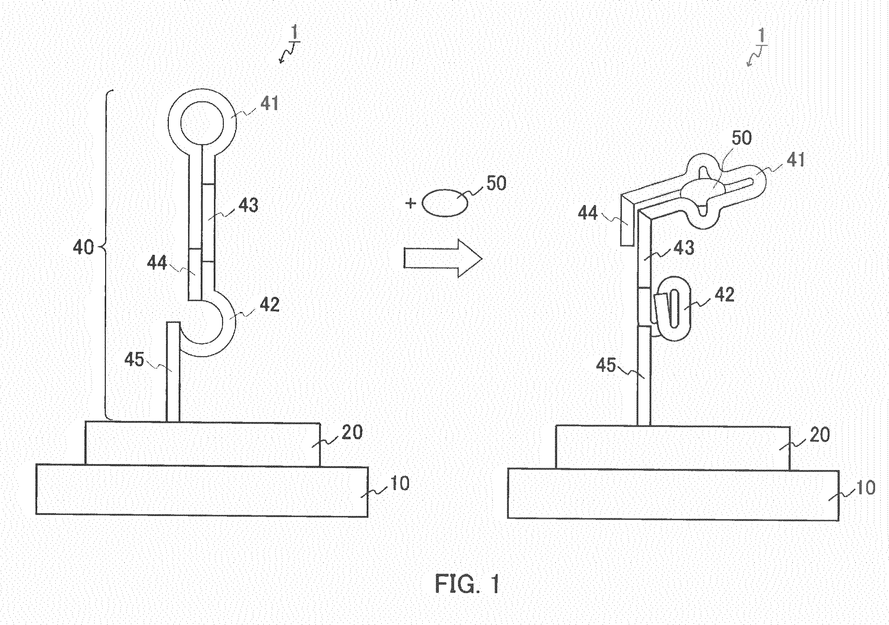

[FIG 1] FIG. 1 shows an explanatory view schematically showing an example of an analytical device in the present invention.

### Description of Embodiments

### Nucleic Acid Element

In the present invention, the nucleic acid element includes a first nucleic acid molecule and a second nucleic acid molecule as mentioned above. The first nucleic acid molecule is a nucleic acid molecule capable of binding to a target. The second nucleic acid molecule is a nucleic acid molecule capable of binding to streptavidin. When the target does not bind to the first nucleic acid molecule, a binding capacity of the second nucleic acid molecule to the streptavidin is inactivated. When the target binds to the first nucleic acid, a binding capacity of the second nucleic acid molecule to the streptavidin is activated.

It is only necessary for the first nucleic acid molecule to be a nucleic acid molecule capable of binding to the target. The first nucleic acid molecule can be selected as appropriate according to the type of the target to be analyzed, for example. Whether or not the first nucleic acid molecule can be bound to the target can be determined by surface plasmon resonance molecular interaction analysis or the like, for example. Specifically, the binding capacity can be detected using Biacore 3000 (trade name, GE Healthcare UK Ltd.) or Biacore X (trade name, GE Healthcare UK Ltd.), for example.

The first nucleic acid molecule may have a secondary structure by self-annealing, for example. The secondary structure can be, for example, a stem-loop structure.

Examples of the first nucleic acid molecule include a single-stranded nucleic acid molecule and a double-stranded nucleic acid molecule.

The first nucleic acid molecule is, for example, a nucleic acid molecule whose structure changes by binding of the target. The change in the structure is, for example, a change in secondary structure. Since the structure of the first nucleic acid molecule is prone to change by binding of the target thereto, the first nucleic acid molecule is, for example, preferably a single-stranded nucleic acid molecule.

Components of the first nucleic acid molecule are not particularly limited, for example, and examples thereof include nucleotide residues. Examples of the nucleotide residues include a ribonucleotide residue and a deoxyribonucleotide residue. The nucleotide residues encompass modified nucleotide residues, for example and may be, for example, derivatives of ribonucleotide residue, derivatives of deoxyribonucleotide residue, or the like. The first nucleic acid molecule may be, for example, a nucleic acid molecule composed of only the nucleotide residues or a nucleic acid molecule containing the nucleotide residues. The first nucleic acid molecule may contain, as the nucleotide residues, only ribonucleotide residues, only deoxyribonucleotide residues, or both of them, for example. Specifically, examples of the first nucleic acid molecule include RNA composed of polyribonucleotide, RNA containing polyribonucleotide, DNA composed of deoxyribonucleotide, and DNA containing deoxyribonucleotide.

The modified nucleotide residues can be, for example, nucleotide residues obtained by modifying sugar residues. Examples of the sugar residues include a ribose residue and a deoxyribose residue. A site to be modified in the sugar residue is not particularly limited and can be, for example, the 2' position and/or the 4' position of the sugar residue. Examples of the modification include methylation, fluorination, amination, and thiation. Examples of the modified nucleotide residues include a 2'-methyl pyrimidine residue and 2'-fluoropyrimidine. Specific examples of the modified nucleotide residues include 2'-methyluracil (2'-methylated-uracil nucleotide residue), 2'-methyl cytosine (2'-methylated-cytosine nucleotide residue), 2'-fluorouracil (2'-fluorinated-uracil nucleotide residue), 2'-fluorocytosine (2'-fluorinated-cytosine nucleotide residue), 2'-aminouracil (2'-aminated-uracil-nucleotide residue), 2'-amino cytosine (2'-aminated-cytosine nucleotide residue), 2'-thiouracil (2'-thiated-uracil nucleotide residue), and 2'-thiocytosine (2'-thiated-cytosine nucleotide residue).

Bases in the nucleotide residues may be, for example, natural bases (non-artificial bases) or non-natural bases. Examples of the natural bases include A, C, G, T, U, and the modified bases thereof. Examples of the non-natural bases include modified bases and altered bases, and the non-natural bases preferably have functions similar to the respective natural bases. Examples of the artificial bases having functions similar to the respective natural bases include an artificial base capable of binding to cytosine (c) in place of guanine (g), an artificial base capable of binding to guanine (g) in place of cytosine (c), an artificial base capable of binding to thymine (t) or uracil (u) in place of adenine (a), an artificial base capable of binding to adenine (a) in place of thymine (t), and an artificial base capable of binding to adenine (a) in place of uracil (u). Examples of the modified bases include a methylated base, a fluorinated base, an aminated base, and a thiated base. Specific examples of the modified bases include 2'-methyluracil, 2'-methylcytosine, 2'-fluorouracil, 2'-fluorocytosine, 2'-aminouracil, 2'-aminocytosine, 2-thiouracil, and 2-thiocytosine. In the present invention, bases represented by a, g, c, t, and u encompass, besides the natural bases, the artificial bases having functions similar to the natural bases, for example.

The first nucleic acid molecule may contain an artificial nucleic acid monomer residue as a component, for example. Examples of the artificial nucleic acid monomer residue include PNA (peptide nucleic acid), LNA (Locked Nucleic Acid), and ENA (2'-O, 4'-C-Ethylenebridged Nucleic Acids). Bases in the monomer residue may be the same as mentioned above, for example.

In the case where the first nucleic acid molecule is a single-stranded nucleic acid molecule, examples of the single-stranded nucleic acid molecule include a single-stranded DNA and a single-stranded RNA. In the case where the first nucleic acid molecule is a double-stranded nucleic acid molecule, examples of the double-stranded nucleic acid molecule include a double-stranded DNA, a double-stranded RNA, and a double-strand of DNA-RNA.

The first nucleic acid molecule may have a natural nucleic acid sequence or a synthesized nucleic acid sequence, for example. A method for synthesizing the first nucleic acid molecule is not particularly limited and can be, for example, a method in which a first nucleic acid is chemically synthesized from the end thereof by a nucleic acid synthesizer such as a DNA synthesizer or an RNA synthesizer using nucleotide such as NTP and dNTP.

The first nucleic acid molecule is, for example, preferably an aptamer. The aptamer generally means a nucleic acid molecule capable of specifically binding to a specific target. The aptamer may be, for example, as mentioned above, any of DNA, RNA, a single-stranded nucleic acid molecule such as a single-stranded RNA or a single-stranded DNA, and a double-stranded nucleic acid molecule such as a double-stranded RNA or a double-stranded DNA.

The length of the first nucleic acid molecule is not particularly limited. The length is not particularly limited, the lower limit thereof is, for example, 7 mer, and the upper limit thereof is, for example, 120 mer. The length is preferably 80 mer, more preferably 35 mer, yet more preferably 20 mer. The range of the length of the first nucleic acid molecule is, for example, from 7 to 120 mer, preferably from 7 to 80 mer, more preferably from 7 to 35 mer, and yet more preferably from 7 to 20 mer.

As mentioned above, the first nucleic acid molecule can be selected as appropriate according to the type of a target to be analyzed and is not at all limited.

A method for producing an aptamer is not particularly limited, and for example, an aptamer can be produced by the above-mentioned known synthesis method. Furthermore, an aptamer capable of binding to a specific target can be produced by the known SELEX (Systematic Evolution of Ligands by Exponential Enrichment) method or the like, for example.

Production of an aptamer by the SELEX method is not particularly limited and can be performed as follows, for example. First, a nucleic acid pool containing a plurality of nucleic acid molecules is provided. Then, nucleic acid molecules in the nucleic acid pool are bound to (associated with) a target, so that complexes between the nucleic acid molecules and the target are formed. Thereafter, nucleic acid molecules involved in formation of the complexes among the obtained complexes are collected. Thus, aptamers capable of specifically binding to the target can be produced.

The second nucleic acid molecule is a nucleic acid molecule capable of binding to streptavidin. When the target does not bind to the first nucleic acid molecule, a binding capacity of the second nucleic acid molecule to the streptavidin is inactivated. When the target binds to the first nucleic acid molecule, a binding capacity of the second nucleic acid molecule to the streptavidin is activated.

In the present invention, "capable of binding to streptavidin" may be capable of binding to any of streptavidin, a fragment of streptavidin, and a derivative of streptavidin, for example. When streptavidin forms a complex with another substance, the second nucleic acid molecule can bind to the streptavidin in the complex, for example.

Whether or not the second nucleic acid molecule can bind to streptavidin can be determined by surface plasmon resonance molecular interaction analysis or the like as mentioned above, for example.

The second nucleic acid molecule may have a secondary structure by self-annealing, for example. The secondary structure can be, for example, a stem-loop structure.

Examples of the second nucleic acid molecule include a single-stranded nucleic acid molecule and a double-stranded nucleic acid molecule.

The second nucleic acid molecule is, for example, a nucleic acid molecule whose structure changes by the change in structure of the first nucleic acid molecule. The change in structure of the second nucleic acid molecule is, for example, a change in secondary structure. Since the structure of the second nucleic acid molecule is prone to change by the change in structure of the first nucleic acid molecule, the second nucleic acid molecule is, for example, preferably a single-stranded nucleic acid molecule.

Components of the second nucleic acid molecule are not particularly limited, for example. The components are, for example, the same as those shown for the first nucleic acid molecule. The second nucleic acid molecule may be, for example, a nucleic acid molecule composed of only the nucleotide residues or a nucleic acid molecule containing the nucleotide residues. The second nucleic acid molecule may contain, as the nucleotide residues, only ribonucleotide residues, only deoxyribonucleotide residues, or both of them, for example. Specifically, examples of the second nucleic acid molecule include RNA composed of polyribonucleotide, RNA containing polyribonucleotide, DNA composed of deoxyribonucleotide, and DNA containing deoxyribonucleotide.

In the case where the second nucleic acid molecule is a single-stranded nucleic acid molecule, examples of the single-stranded nucleic acid molecule include a single-stranded DNA and a single-stranded RNA. In the case where the second nucleic acid molecule is a double-stranded nucleic acid molecule, examples of the double-stranded nucleic acid molecule include a double-stranded DNA, a double-stranded RNA, and a double-strand of DNA-RNA.

The second nucleic acid molecule may have a natural nucleic acid sequence or a synthesized nucleic acid sequence, for example. A method for synthesizing the second nucleic acid molecule is not particularly limited and is, for example, the same as shown for the first nucleic acid molecule.

The second nucleic acid molecule is, for example, preferably an aptamer. The aptamer may be, for example, as mentioned above, any of DNA, RNA, a single-stranded nucleic acid molecule such as a single-stranded RNA or a single-stranded DNA, and a double-stranded nucleic acid molecule such as a double-stranded RNA or a double-stranded DNA.

The length of the second nucleic acid molecule is not particularly limited. The length is not particularly limited, the lower limit thereof is, for example, 7 mer, and the upper limit thereof is, for example, 120 mer. The length is preferably 80 mer, more preferably 35 mer, yet more preferably 20 mer. The range of the length of the second nucleic acid molecule is, for example, from 7 to 120 mer, preferably from 7 to 80 mer, more preferably from 7 to 35 mer, and yet more preferably from 7 to 20 mer.

For example, the second nucleic acid molecule preferably contains any of the following polynucleotides (a) to (d):
(a) a polynucleotide composed of a base sequence represented by any of SEQ ID NOs: 1 to 10;
(b) a polynucleotide composed of a base sequence obtained by displacement, deletion, addition, and/or insertion of one or more bases in the base sequence (a) and capable of binding to streptavidin;
(c) a polynucleotide composed of a base sequence having a identity of 50% or more to the base sequence (a) and capable of binding to streptavidin; and
(d) a polynucleotide composed of a base sequence which hybridizes with the base sequence (a) under stringent conditions or a base sequence complementary thereto and capable of binding to streptavidin.

The base sequences of SEQ ID NOs: 1 to 10 are shown below. In each of the base sequences, underlined bases indicate a common sequence.
SEQ ID NO: 1 (85)
   taatacgact cactatagca atggtacggt acttcccgac gcaccgatcg caggttcggg acaaaagtgc acgctacttt gctaa
SEQ ID NO: 2 (18)
   AC GCACCGATCG CAGGTT
SEQ ID NO: 3 (20)
   GAC GCACCGATCG CAGGTTC
SEQ ID NO: 4 (22)
   CGAC GCACCGATCG CAGGTTCG
SEQ ID NO: 5 (24)
   CCGAC GCACCGATCG CAGGTTCGG
SEQ ID NO: 6 (26)
   CCCGACGCACCGATCG CAGGTTCGGG
SEQ ID NO: 7 (28)
   TCCCGAC GCACCGATCG CAGGTTCGGG A
SEQ ID NO: 8 (60_3-10)
   GCAATGGTACGGT ACTTCCCGAC GCACCGATCG CAGGTTCGGG ACAAAAG
SEQ ID NO: 9 (60_5-10)
   GGT ACTTCCCGAC GCACCGATCG CAGGTTCGGG ACAAAAGTGC ACGCTAC

SEQ ID NO: 10 (60)
GCA ATGGTACGGT ACTTCCCGAC GCACCGATCG CAGGTTCGGG ACAAAAGTGC
ACGCTAC

Among the base sequences, a base sequence of any of SEQ ID NOs: 2 to 10 is preferable because the base sequence has superior binding property to streptavidin, and the second nucleic acid molecule can be easily designed by the base sequence, for example.

The second nucleic acid molecule may be, for example, a nucleic acid molecule containing a polynucleotide composed of the base sequence or a nucleic acid molecule composed of the polynucleotide.

Hereinafter, a nucleic acid molecule composed of the polynucleotide (a) or a nucleic acid molecule containing the polynucleotide (a) is referred to as a nucleic acid molecule (a). A nucleic acid molecule composed of the polynucleotide (b) or a nucleic acid molecule containing the polynucleotide (b) is referred to as a nucleic acid molecule (b). A nucleic acid molecule composed of the polynucleotide (c) or a nucleic acid molecule containing the polynucleotide (c) is referred to as a nucleic acid molecule (c). A nucleic acid molecule composed of the polynucleotide (d) or a nucleic acid molecule containing the polynucleotide (d) is referred to as a nucleic acid molecule (d).

The nucleic acid molecule (a) is, for example, a nucleic acid molecule composed of the polynucleotide (a) or a nucleic acid molecule containing the polynucleotide (a), (a) a polynucleotide composed of a base sequence represented by any of SEQ ID NOs: 1 to 10.

The nucleic acid molecule (a) is, for example, a nucleic acid molecule capable of forming a structure represented by the following general formula (1). The nucleic acid molecule is, for example, a nucleic acid molecule satisfying the general formula (1) and composed of or containing a base sequence of SEQ ID NO: 1 or a partial sequence thereof.

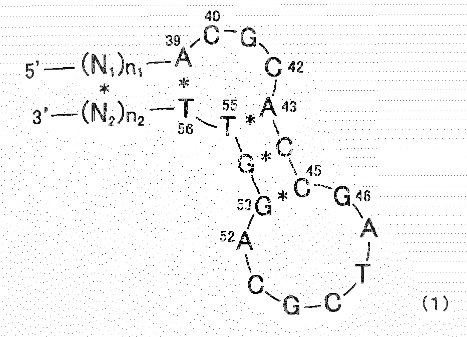

In the general formula (1), the numeral beside each base indicates the position of the base in the base sequence of SEQ ID NO: 1 and is not necessary to indicate the position of the base in the nucleic acid molecule represented by the general formula (1). In the general formula (1), each asterisk indicates a hydrogen bond and shows being capable of forming a stem structure. In the general formula (1), each of N₁ and N₂ indicates a nucleotide residue. In the general formula (1), n₁ indicates the number of the nucleotide residues N₁, and n₂ indicates the number of the nucleotide residues N₂. (N₁)n₁ and (N₂)n₂ can form a stem structure by mutually forming a hydrogen bond. In the case where (N₁)n₁ has a plurality of nucleotide residues, the nucleotide residues may be identical to or different from each other. In the case where (N₂)n₂ has a plurality of nucleotide residues, the nucleotide residues may be identical to or different from each other.

As shown in the general formula (1), in the base sequence represented by SEQ ID NO: 1, a sequence of bases at positions 40 to 42 is capable of forming a bulge structure, a sequence of bases at positions 46 to 52 is capable of forming a loop structure, and a sequence of bases at positions 43 to 45 and a sequence of bases at positions 53 to 55 are capable of forming a stem structure, for example. As shown in the general formula (1), in a base sequence represented by SEQ ID NO: 1 of the nucleic acid molecule, a sequence "(N₁)n₁-A" upstream (5' side) from a base at position 39 and a sequence "T-(N₂)n₂" downstream (3' side) from a base at position 56 can form a stem structure.

In the present invention, "capable of forming a loop structure" encompasses actually forming a loop structure and being capable of forming a loop structure according to conditions even though a loop structure is not formed, for example. Moreover, "capable of forming a loop structure" further encompasses both of the case of checking experimentally and the case of predicting by a computer simulation, for example. In the present invention, "capable of forming a stem structure" encompasses actually forming a stem structure and being capable of forming a stem structure according to conditions even through a stem structure is not formed, for example. Moreover, "capable of forming a stem structure" further encompasses both of the case of checking experimentally and the case of predicting by a computer simulation, for example.

In the general formula (1), n₁ and n₂ are each 0, 1, 2, 3, 4, or 5 and are identical to each other, for example.
In the case where n₁ and n₂ are 0, "(N₁)n₁-A" is "A", "T-(N₂)n₂" is "T", the sequence of the general formula (1) is represented by SEQ ID NO: 2, and the stem structure has one base pair, for example.
In the case where n₁ and n₂ are 1, "(N₁)n₁-A" is "GA", "T-(N₂)n₂" is "TC", the sequence of the general formula (1) is represented by SEQ ID NO: 3, and the stem structure has two base pairs, for example.
In the case where n₁ and n₂ are 2, "(N₁)n₁-A" is "CGA", "T-(N₂)n₂" is "TCG", the sequence of the general formula (1) is represented by SEQ ID NO: 4, and the stem structure has three base pairs, for example.
In the case where n₁ and n₂ are 3, "(N₁)n₁-A" is "CCGA", "T-(N₂)n₂" is "TCGG", the sequence of the general formula (1) is represented by SEQ ID NO: 5, and the stem structure has four base pairs, for example.
In the case where n₁ and n₂ are 4, "(N₁)n₁-A" is "CCCGA", "T-(N₂)n₂" is "TCGGG", the sequence of the general formula (1) is represented by SEQ ID NO: 6, and the stem structure has five base pairs, for example.
In the case where n₁ and n₂ are 5, "(N₁)n₁-A" is "TCCCGA", "T-(N₂)n₂" is "TCGGGA", the sequence of the general formula (1) is represented by SEQ ID NO: 7, and the stem structure has six base pairs, for example.

The nucleic acid molecule (a) may be, for example, as mentioned above, a nucleic acid molecule having a structure represented by the general formula (1) or a nucleic acid molecule containing a structure represented by the general formula (1). In the latter case, for example, a sequence may further be added on any or both of the upstream side of (N₁) n₁ and the downstream side of (N₂) n₂ in the general formula (1). The additional sequence can be set on the basis of the base sequence represented by SEQ ID NO: 1, and the structure formed by the additional sequence is not particularly limited, for example.

The nucleic acid molecule (b) is, for example, as mentioned above, a nucleic acid molecule containing the polynucleotide (b) or a nucleic acid molecule composed of the polynucleotide (b), (b) a polynucleotide composed of a base sequence obtained by displacement, deletion, addition, and/or insertion of one or more bases in the base sequence (a) and capable of binding to streptavidin.

In the base sequence (a), "one or more" is not particularly limited and is, for example, 1 to 5, preferably 1 to 4, more preferably 1 to 3, yet more preferably 1 or 2, and particularly preferably 1.

The nucleic acid molecule (c) is, for example, as mentioned above, a nucleic acid molecule containing the polynucleotide (c) or a nucleic acid molecule composed of the polynucleotide (c), (c) a polynucleotide composed of a base sequence having a identity of 50% or more to the base sequence (a) and capable of binding to streptavidin.

In the polynucleotide (c), the identity (homology) is, for example, 60% or more, preferably 70% or more, more preferably 80% or more, yet more preferably 90% or more, further more preferably 95% or more, particularly preferably 99% or more. The identity can be calculated using the BLAST or the like under default conditions, for example.

The nucleic acid molecule (d) is, for example, as mentioned above, a nucleic acid molecule containing the polynucleotide (d) or a nucleic acid molecule composed of the polynucleotide (d), (d) a polynucleotide composed of a base sequence which hybridizes with the base sequence (a) under stringent conditions or a base sequence complementary thereto and capable of binding to streptavidin.

In the polynucleotide (d), "hybridization under stringent conditions" means hybridization under experimental conditions well known to those skilled in the art, for example. Specifically, the "stringent conditions" refer to conditions under which the base sequence can be identified after conducting hybridization at 60°C to 68°C in the presence of 0.7 to 1 mol/l NaCl and then washing at 65°C to 68°C using a 0.1- to 2-fold SSC solution, for example. Note here that 1 x SSC is composed of 150 mmol/L NaCl and 15 mmol/L sodium citrate.

The full length of each of the nucleic acid molecules (b), (c), and (d) is, for example, from 18 to 60 mer, preferably from 18 to 50 mer, and specifically 18 mer, 20 mer, 22 mer, 24 mer, 26 mer, 28 mer, or 50 mer.

In the case where the second nucleic acid molecule is the double-stranded nucleic acid molecule, the second nucleic acid molecule can be, for example, a double-stranded nucleic acid molecule of any of single-stranded polynucleotides (a) to (d) and any of single-stranded polynucleotides composed of the respective base sequences complementary thereto. In the case where the second nucleic acid molecule is the double-stranded nucleic acid molecule, the double-stranded nucleic acid molecule may be caused to be single-stranded nucleic acid molecules by denaturation or the like prior to the use thereof, for example.

The second nucleic acid molecule can be produced by a known method based on information on the above-mentioned base sequences, for example. The known method is not particularly limited, and examples thereof include a chemical synthesis method using an automatic synthesis device, a synthesis method by an enzyme reaction using various polymerases, and a synthesis by in vitro transcription from a DNA template.

The second nucleic acid molecule can be produced by a method in which complexes between a nucleic acid molecule pool and streptavidin is formed using the nucleic acid molecule pool and the streptavidin, and candidates of nucleic acid molecules involved in formation of the complexes are selected, for example. Examples of such method include a method according to the SELEX method and equivalent methods thereof and a method in which complexes are formed using a carrier on which streptavidin is immobilized, and thereafter candidates of nucleic acid molecules involved in formation of the complexes are collected. Examples of the carrier include agarose gel and polyacrylamide gel.

The second nucleic acid molecule may be produced by partially altering the nucleic acid molecule obtained by the above-mentioned method, for example. In this case, for example, the nucleic acid molecule can be altered with reference to the result obtained using a method for predicting a secondary structure based on the base sequence of the obtained nucleic acid molecule. The method for predicting a secondary structure is not particularly limited and can be, for example, a method in which candidates of the secondary structure of a nucleic acid molecule are searched for, and secondary structures which are energetically stable are predicted among the candidates of the secondary structure. The prediction of a secondary structure may be, for example, a prediction of a secondary structure based on minimizing energy functions of candidates of the secondary structure obtained by dividing the base sequence of the nucleic acid molecule into a stem region composing a base pair of such as a Watson-Crick type and a single-strand region such as a loop structure composed of bases other than the stem region.

The prediction of a secondary structure based on minimizing energy functions of candidates of the secondary structure is described below. First, candidates of bases composing a base pair of such as a Watson-Crick type and candidates of a single-strand region other than these candidates of the base pair are searched in the base sequence of the target nucleic acid molecule. Then, candidates of the secondary structure are identified through excluding theoretically impossible combinations such as a combination in which bases composing a candidate of the base pair overlap with bases composing a candidate of the single-strand region and the like from all combinations of candidates of the base pair and the candidates of the single-strand region searched for. Energy functions of the candidates of the secondary structure are calculated, and the secondary structure with the minimum energy function is searched for from the identified candidates of the secondary structure. At that time, a method for calculating an energy function of a candidate of the secondary structure may be a method in which based on free energy of each stem region and each single-strand region composing a candidate of the secondary structure, this free energy of the candidate of the secondary structure is used as an energy function of the candidate of the secondary structure. The secondary structure with the minimum energy function among the candidates of the secondary structure identified as mentioned above is used as a secondary structure of the base sequence of the target nucleic acid molecule.

The second nucleic acid molecule may be altered by replacement or deletion of a base composing a characteristic site in the secondary structure or insertion or addition of a base to a characteristic part in the secondary structure with reference to the result of the secondary structure obtained as mentioned above. For example, some of bases composing a stem region and/or a single-strand region of the secondary structure may be replaced using the nucleic acid molecule provided as mentioned above as a parent molecule. Some of bases composing a stem region and/or a single-strand region of the secondary structure may be deleted. The lengths of the stem region and/or a single-strand region may be shortened and/or extended by inserting or adding a single base or a plurality of bases to a stem region and/or a single-strand region of the secondary structure.

In the case where the first nucleic acid molecule and/or the second nucleic acid molecule is RNA, the RNA may preferably have a ribonuclease resistance, for example. A method for making the nucleic acid molecule having a ribonuclease resistance is not particularly limited. Specifically, examples of the method include a method in which a part of a ribonucleotide residue composing RNA is modified by methylation or the like and a method in which a part or whole of the ribonucleotide residue is made into deoxyribonucleotide (DNA) or LNA. In the case where the nucleic acid molecule is RNA containing the deoxyribonucleotide residue, the site of the deoxyribonucleotide residue is not particularly limited and is, for example, preferably a region capable of forming a stem structure. In the case where the nucleic acid molecule is RNA containing a monomer residue such as the LNA, the site of the monomer residue is not particularly limited and is, for example, preferably a region capable of forming a stem structure. Other than these, the method can be a method in which PEG (polyethyleneglycol) with a few tens of kilodaltons or deoxythymidine is bound to the 5' end and/or the 3' end of the first nucleic acid molecule and/or the second nucleic acid molecule.

The first nucleic acid molecule and/or the second nucleic acid molecule may be, for example, a modified nucleotide residue obtained by modifying a part of the nucleotide residue as mentioned above. The site of the modified nucleotide residue in the nucleic acid molecule is not particularly limited and is, for example, preferably the end of the region capable of forming a stem structure and the end of the region capable of forming a loop structure or a bulge structure.

As mentioned above, the nucleic acid element contains the first nucleic acid molecule and the second nucleic acid molecule in the present invention. The nucleic acid element may be, for example, an element composed of only the first nucleic acid molecule and the second nucleic acid molecule or may further include another component. The another component can be, for example, a linker.

Components of the linker are not particularly limited and are the same as those for the first nucleic acid molecule and the second nucleic acid molecule mentioned above, for example. The linker may be, for example, a nucleic acid molecule composed of only the nucleotide residue or a nucleic acid molecule containing the nucleotide residue. The linker may be, for example, a single-stranded nucleic acid molecule or a double-stranded nucleic acid molecule. In the case where the linker is a single-stranded nucleic acid molecule, examples thereof include a single-stranded DNA and a single-stranded RNA. In the case where the linker is a double-stranded nucleic acid molecule, examples thereof include a double-stranded DNA, a double-stranded RNA, and a double strand of DNA-RNA. The length of the linker is not particularly limited.

In the nucleic acid element, the first nucleic acid molecule and the second nucleic acid molecule are preferably joined to each other, and for example, one end of the first nucleic acid molecule is joined to one end of the second nucleic acid molecule. The 5' end of the first nucleic acid molecule may be joined to the 3' end of the second nucleic acid molecule, or the 3' end of the first nucleic acid molecule may be joined to the 5' end of the second nucleic acid molecule, for example. The former is preferred.

The first nucleic acid molecule and the second nucleic acid molecule may be joined to each other directly or indirectly, for example.

In the case of the direct joining, one end of the first nucleic acid molecule and one end of the second nucleic acid molecule are joined to each other by phosphodiester binding, for example. Specifically, the direct binding can be, for example, joining of the 5' end of the first nucleic acid molecule to the 3' end of the second nucleic acid molecule by phosphodieter binding or joining of the 3' end of the first nuclei acid molecule to the 5' end of the second nucleic acid molecule by phosphodiester binding. The former is preferred.

In the case of the indirect joining, the first nucleic acid molecule and the second nucleic acid molecule are joined to each other via the linker, for example. Hereinafter, a linker intervening between the first nucleic acid molecule and the second nucleic acid molecule is referred to as an intervening linker or an intervening sequence. The intervening linker can be in the form in which one end is joined to one end of the first nucleic acid molecule, and the other end is joined to one end of the second nucleic acid molecule, for example. Specifically, the indirect joining is, for example, joining of one end of the intervening linker to the 5' end of the first nucleic acid molecule and joining of the other end to the 3' end of the second nucleic acid molecule or may be joining of one end to the 3' end of the first nucleic acid molecule and joining of the other end to the 5' end of the second nucleic acid molecule. The former is preferred. The joining of the first nuclei acid molecule or the second nucleic acid molecule to the linker can be, for example, joining by phosphodiester binding.

The nucleic acid element may further have the linker on any of the end sides thereof. Hereinafter, this linker is referred to as an additional linker or an additional sequence. The nucleic acid element may have the linker at the end opposite to the end to which the second nucleic acid molecule is joined in the first nucleic acid molecule or at the end opposite to the end to which the first nucleic acid molecule is joined in the second nucleic acid molecule, for example. The nucleic acid element may have the additional linker at each of the opposite end in the first nucleic acid molecule and the opposite end in the second nucleic acid molecule.

The full length of the nucleic acid element is not particularly limited and can be set as appropriate according to the above mentioned lengths of the first nucleic acid molecule and the second nucleic acid molecule and the length of a linker, for example. In the nucleic acid element, the lengths of the first nucleic acid molecule and the second nucleic acid molecule may be identical to or different from each other.

The nucleic acid element is, for example, preferably a single-stranded nucleic acid molecule obtained by joining the first nucleic acid molecule and the second nucleic acid molecule to each other. In the case where the nucleic acid element is a single-stranded nucleic acid molecule, the structure of the first nucleic acid molecule changes by binding of a target to the first nucleic acid molecule, and this change easily cause the change in structure of the second nucleic acid molecule, for example. The change in structure is, for example, as mentioned above, the change in secondary structure.

A method for designing the nucleic acid element is not particularly limited as long as the first nucleic acid molecule and the second nucleic acid molecule are joined to each other in the nucleic acid molecule, for example. Specifically, the nucleic acid element is designed as follows. The first nucleic acid molecule and the second nucleic acid molecule are joined to each other so that when the target does not bind to the first nucleic acid molecule, a binding capacity of the second nucleic acid molecule to the streptavidin is inactivated, and when the target binds to the first nucleic acid molecule, a binding capacity of the second nucleic acid molecule to the streptavidin is activated.

It is preferred that a binding capacity of the second nucleic acid molecule to the streptavidin is controlled in the nucleic acid element as follows, for example. That is, in the nucleic acid element, the binding capacity of the second nucleic acid molecule to the streptavidin is inactivated by caging the second nucleic acid molecule in the state where the target does not bind to the first nucleic acid molecule, for example. Further, the binding capacity of the second nucleic acid molecule to the streptavidin is activated by self-association through binding the target to the first nucleic acid molecule.

Specifically, the nucleic acid element can be in the following form, for example. In the state where the target does not bind to the first nucleic acid molecule, a part of the first nucleic acid molecule and a part of the second nucleic acid molecule form a stem structure in the nucleic acid element, the second nucleic acid molecule is caged by the stem structure, and the first nucleic acid molecule forms a stem-loop structure to be a site to which the target binds, for example. On the other hand, the stem structure of the part of the first nucleic acid molecule and the part of the second nucleic acid molecule is released by binding the target to the first nucleic acid molecule, and thus releasing the caging of the second nucleic acid molecule, and the binding capacity of the second nucleic acid molecule to the streptavidin is activated by self-association of the second nucleic acid molecule.

In the case where the nucleic acid element includes the intervening linker and the additional linker, it is preferred that the binding capacity of the second nucleic acid molecule to the streptavidin is controlled as follows, for example. In the nucleic acid element, one end of the intervening linker is joined to the 5' end of the first nucleic acid molecule, the other end is joined to the 3' end of the second nucleic acid molecule, and the additional linker is joined to the 3' end of the first nucleic acid molecule, for example. In the state where the target does not bind to the first nucleic acid molecule in the nucleic acid element, for example, the intervening linker and the 3' end region of the first nucleic acid molecule form a stem structure, and the additional linker and the 3' end region of the second nucleic acid molecule form a stem structure. Then the second nucleic acid molecule is caged by the stem structures, and thus, the binding capacity of the second nucleic acid molecule to the streptavidin is inactivated. Further, the first nucleic acid molecule forms a stem-loop structure to be a site to which the target binds. On the other hand, the stem structure with the intervening linker and the stem structure with the additional linker are released by binding of the target to the first nucleic acid molecule. Thus, the caging of the second nucleic acid molecule is released, and the binding capacity of the second nucleic acid molecule to the streptavidin is activated by self-association of the second nucleic acid molecule.

The nucleic acid element may be obtained by producing the first nucleic acid molecule and the second nucleic acid molecule and then binding them to each other or may be obtained by designing a sequence in which they are joined to each other and then synthesizing the nucleic acid molecule on the basis of the sequence, for example. At that time, the sequence may be modified through predicting the secondary structure by a computer or the like, for example.

### Analytical device

The analytical device according to the present invention is, as mentioned above, an analytical device including: a basal plate; a nucleic acid element; and a detection section of detecting a signal, wherein the nucleic acid element and the detection section are arranged on the basal plate, the nucleic acid element includes a first nucleic acid molecule and a second nucleic acid molecule, the first nucleic acid molecule is a nucleic acid molecule capable of binding to a target, the second nucleic acid molecule is a nucleic acid molecule capable of binding to streptavidin, when the target does not bind to the first nucleic acid molecule, a binding capacity of the second nucleic acid molecule to the streptavidin is inactivated, when the target binds to the first nucleic acid molecule, a binding capacity of the second nucleic acid molecule to the streptavidin is activated, and the detection section detects binding between the second nucleic acid molecule and the streptavidin. In the present invention, the nucleic acid element is the same as mentioned above.

It is only necessary for the detection section to detect binding between the second nucleic acid molecule and the streptavidin. Specifically, for example, it is only necessary for the detection section to detect a signal derived from the binding. Examples of the signal include an optical signal such as a chromogenic signal or a luminescent signal and an electrical signal.

As mentioned below, it is preferred that labeled streptavidin obtained by labeling streptavidin with a labeling substance is used as streptavidin in analysis using the analytical device. The signal derived from the binding is, for example, preferably, a signal derived from the labeling substance.

The labeling substance preferably is a labeling substance which emits a signal. The labeling substance may be, for example, a labeling substance which emits a signal independently or indirectly. In the former case, examples of the labeling substance include fluorophore and radioisotope, and a signal thereof can be detected by the method performed under the conditions according to the type of the labeling substance. In the latter case, the labeling substance can be, for example, an enzyme and is preferably an enzyme which catalyzes an oxidation-reduction reaction. In this case, the signal is, for example, preferably a signal emitted from a substrate by the oxidation-reduction reaction, and specifically, the signal is generated by causing the enzyme to react in the presence of the substrate. It is only necessary for the oxidation-reduction reaction to cause electron transfer between two substrates in the process of generating a product from the substrates, for example. Examples of the enzyme include peroxidase such as horseradish peroxidase, alkaline phosphatase, β-galactosidase, urease, catalase, glucose oxidase, lactate dehydrogenase, and amylase.

The substrate can be decided as appropriate according to the type of the enzyme, for example. In the case where the enzyme is peroxidase, the substrate is not particularly limited, and examples thereof include 3,3',5,5'-Tetramethylbenzidine (TMB), 1,2-Phenylenediamine (OPD), 2,2'-Azinobis(3-ethylbenzothiazoline-6-sulfonic Acid Ammonium Salt (ABTS), 3,3'-Diaminobenzidine (DAB), 3,3'-Diaminobenzidine Tetrahydrochloride Hydrate (DAB4HCl), 3-Amino-9-ethylcarbazole (AEC), 4-Chloro-1-naphthol (4C1N), 2,4,6-Tribromo-3-hydroxybenzoic Acid, 2,4-Dichlorophenol, 4-Aminoantipyrine, 4-Aminoantipyrine Hydrochloride, and luminol.

The analytical device according to the present invention may further include a reagent section containing the substrate for the oxidation-reduction reaction, for example. The reagent section is, for example, preferably arranged in the detection section and more preferably arranged on an electrode system.

In the case where the signal is the electrical signal, the detection section has an electrode system, for example. The electrode system may include a working electrode and a counter electrode or may include a working electrode, a counter electrode, and a reference electrode, for example. The material of the electrode is not particularly limited, and examples thereof include platinum, silver, gold, and carbon. Examples of the working electrode and the counter electrode include a platinum electrode, a silver electrode, a gold electrode, and a carbon electrode. The reference electrode can be, for example, a silver/silver chloride electrode. The silver/silver chloride electrode can be formed by laminating a silver chloride electrode on a silver electrode, for example.

The detection section can be formed by arranging the electrode on the upper surface of the basal plate, for example. A method for arranging the electrode is not particularly limited, a known method can be employed, for example, and specific examples thereof include thin-film forming methods such as an evaporation method, a sputtering method, a screen printing method, and a plating method. The electrode may be arranged directly or indirectly on the basal plate, for example. The indirect arrangement can be, for example, an arrangement via another member (the same applies hereinafter).

As mentioned above, it is only necessary for the nucleic acid element to be arranged on the basal plate. It is preferred that the nucleic acid element is immobilized on the basal plate. The nucleic acid element may be arranged directly or indirectly on the surface of the basal plate, for example. Specifically, for example, the nucleic acid element is arranged preferably on the detection section of the basal plate, more preferably on the electrode in the detection section, and yet more preferably on the working electrode among electrodes. The nucleic acid element may be arranged directly or indirectly on the detection section or the electrode, for example. Hereinafter, the "arrangement or immobilization of the nucleic acid element on the basal plate" encompasses the arrangement or immobilization of the nucleic acid element on the detection section in the basal plate or on the electrode in the detection section unless otherwise shown.

A method for arranging the nucleic acid element is not particularly limited, and a conventionally known method for immobilizing a nucleic acid can be employed. The method for immobilizing a nucleic acid can be, for example, a method for immobilizing a pre-prepared nucleic acid on the basal plate, preferably on the detection section, more preferably on the electrode. This method is, for example, a method utilizing photolithography, and a specific example thereof can be found in references such as U.S. Pat. No. 5,424,186 and the like. Furthermore, a method for immobilizing a nucleic acid can be, for example, a method for synthesizing a nucleic acid element on the basal plate, preferably on the detection section, more preferably on the electrodes. This method can be, for example, a spot method, and a specific example thereof can be found in references such as U.S. Pat. No. 5,807,522 and JP H10-503841 A.

Any of the end side of the first nucleic acid molecule and the end side of the second nucleic acid molecule in the nucleic acid element may be arranged on the basal plate, for example. The end side can be, for example, one end of the first nucleic acid molecule or the second nucleic acid molecule in the nucleic acid element. In the case where the first nucleic acid molecule has an additional linker, the end side may be, for example, the end of the additional linker, opposite to the end to which the first nucleic acid molecule is linked. On the other hand, when the second nucleic acid molecule has an additional linker, the end side may be, for example, the end of the additional linker, opposite to the end to which the second nucleic acid molecule is linked, and this form is preferred.

The analytical device according to the present invention may include only one type of the nucleic acid element or two or more types of the nucleic acid elements each of whose targets are different, for example. It is preferred in the latter case that each of the nucleic acid elements has a first nucleic acid that can bind to a different target. As described above, when the analytical device according to the present invention includes two or more types of the nucleic acid elements each of whose targets are different, it becomes possible to detect two or more types of targets by one analytical device, for example. In the case where the analytical device according to the present invention includes two or more types of the nucleic acid elements, it is preferred that the analytical device includes a plurality of detection sections, and different nucleic acid elements are arranged in the respective detection sections. Such analytical device can be formed by, for example, fractionating the surface of the basal plate into matrixes, forming the above-mentioned electrode system in the respective fraction regions, and arranging nucleic acid elements in the respective detection sections. In the analytical device according to the present invention, the number of the nucleic acid elements arranged in one detection section is not particularly limited.

The basal plate is not particularly limited and is, for example, preferably a basal plate having an insulating surface. The basal plate may be, for example, a basal plate composed of an insulating material or a basal plate having an insulating layer composed of an insulating material on the surface thereof. The insulating material is not particularly limited, and examples thereof include known materials such as glass, ceramics, an insulating plastic, and paper. The insulating plastic is not particularly limited, and examples thereof include a silicone resin, a polyimide resin, an epoxy resin, and a fluorine resin.

A target to be analyzed by an analytical device according to the present invention is not particularly limited. Examples of the target include a high-molecular compound, a low-molecular compound, an organic compound, and an inorganic compound. Examples of the high molecular compound or the organic compound include microorganisms, virus, polysaccharide, protein, nucleic acid, and a resin. Examples of the low-molecular compound include a pesticide, a pharmaceutical, a chemical agent, oligosaccharide, monosaccharide, lipid, oligopeptide, amino acid, vitamins, and a biologically active substance. Examples of the inorganic compound include minerals, mineral acid, and metals.

A sample to be analyzed in the present invention is not particularly limited, and examples thereof include food, a pharmaceutical, a chemical agent, the ground, an animal, a plant, a microorganism, a virus, water, refuse, and a waste. Examples of the food include foodstuffs and drinks. Examples of the water include tap water, drain water, river water, seawater, rainwater, and snow.

Next, the analytical device according to the present invention is described with reference to a specific example. Note here that the present invention is not limited by the following example.

FIG 1 shows a schematic view of an example of an analytical device according to the present invention. As shown in FIG. 1, an analytical device 1 includes a basal plate 10, an electrode 20, and a nucleic acid element 40, the electrode 20 is arranged on the basal plate 10, and the nucleic acid element 40 is immobilized on the electrode 20. On the basal plate 10, a region in which the electrode 20 is arranged is a detection section. The nucleic acid element 40 is a single-stranded nucleic acid molecule composed of a first nucleic acid molecule 41, a second nucleic acid molecule 42, an intervening linker 43, a first additional linker 44, and a second additional linker 45. In the nucleic acid element 40, the first nucleic acid molecule 41 and the second nucleic acid molecule 42 are joined to each other via the intervening linker 43, the first additional linker 44 is joined to the end of the first nucleic acid molecule 41, and the second additional linker 45 is joined to the end of the second nucleic acid molecule 42. The nucleic acid element 40 is immobilized on the electrode 20 via the second additional linker 45 joined to the second nucleic acid molecule 42. The first nucleic acid molecule 41 is preferably a single-strand and is preferably form a stem-loop structure by self-annealing in the state of not binding to a target as shown in the left figure of FIG. 1.

A method for using an analytical device 1 is described below with reference to an example using an first nucleic acid molecule 41 as an aptamer capable of binding to a target 50 and using a second nucleic acid molecule 42 as DNA capable of binding to labeled streptavidin obtained by labeling streptavidin with peroxidase.

First, a sample is added to a detection section of the analytical device 1. When a target 50 is not present in the sample, the target does not bind to the first nucleic acid molecule 41 of the nucleic acid element 40 as shown in the left figure of FIG 1, so that the second nucleic acid molecule 42 does not bind to streptavidin. Specifically, the second nucleic acid molecule 42 and a first additional liner 44 form a stem structure, so that the second nucleic acid molecule 42 is caged, and a binding capacity of the second nucleic acid molecule 42 to streptavidin is inactivated. Thus, there is no electron transfer caused by peroxidase, so that an electrical signal cannot be detected by the electrode 20 of the detection section. On the other hand, when the target 50 is present in the sample, the target binds to the first nucleic acid molecule 41 of the nucleic acid element 40 as shown in the right figure of FIG. 1, so that the structure of the second nucleic acid molecule 42 changes to a secondary structure capable of binding to streptavidin. Specifically, when the target 50 binds to the first nucleic acid molecule 41, the structure of the first nucleic acid molecule 41 changes, so that the stem structure of the second nucleic acid molecule 42 and the first additional linker 44 is released. Thus, the second nucleic acid molecule 42 is self-associated, and a binding capacity of the second nucleic acid molecule 42 to streptavidin is activated.

Then, the labeled streptavidin is added to the detection section, so that the labeled streptavidin is bound to the second nucleic acid molecule 42 whose binding capacity is activated. Thereafter, the detection section is washed, so that the labeled streptavidin which is not bound to the second nucleic acid molecule 42 is removed. Subsequently, a substrate is added to the detection section so as to perform an enzyme reaction by peroxidase in the labeled streptavidin. Electrons are transferred in the process of generating a product from the substrate by the peroxidase. Thus, an electrical signal can be detected by the electrode 20 in the detection section. As described above, according to the analytical device 1, the presence or absence of the target in the sample can be analyzed through detecting an electrical signal.

The order of adding the sample and the labeled streptavidin is not particularly limited, and they may be added at the same time, the labeled streptavidin may be added after adding the sample, or the sample may be added after adding the labeled streptavidin, for example. The order of adding the substrate is not particularly limited, and the substrate is preferably added after adding the sample and the labeled streptavidin, for example.

### Analysis method

The analytical method according to the present invention is, as mentioned above, an analytical method for analyzing a target using the analytical device according to the present invention, the analytical method including: an adding step of adding a sample and streptavidin to the analytical device; and a detecting step of detecting binding between the second nucleic acid molecule and the streptavidin in the detection section, whereby detecting a target.

The analytical method according to the present invention can be performed in the same manner as described for the analytical device according to the present invention, for example.

The order of adding the sample and the streptavidin is not particularly limited. The sample and the streptavidin may be added at the same time, the streptavidin may be added after adding the sample, or the sample may be added after adding the streptavidin, for example. The streptavidin is preferably a labeled streptavidin obtained by binding the labeling substance to streptavidin.

The labeling substance is, for example, as mentioned above, a labeling substance which emits a signal independently or indirectly. The latter labeling substance preferably is an enzyme which catalyzes the oxidation-reduction reaction, and the detecting step is preferably performed in the presence of a substrate for the oxidation-reduction reaction, for example.

The detecting step is, as mentioned above, a detecting step of detecting binding between the second nucleic acid molecule and the streptavidin, whereby detecting a target. The binding between the second nucleic acid molecule and the streptavidin can be detected by detecting a signal derived from the labeling substance, for example. The detection of the signal can be decided as appropriate according to the type of the labeling substance, for example. Examples of the detection include electrochemical detection of detecting the electrical signal and optical detection of detecting an optical signal. The optical signal may be, for example, a signal of the labeling substance itself or a signal generated by an enzyme reaction caused by the labeling substance in the presence of the substrate. The substrate is not particularly limited and preferably is a substrate which develops a color or emits light by the enzyme reaction. The electrochemical detection can be, for example, detection of an electrochemical signal and can be carried out by measuring signal intensity such as current. The electrochemical signal is generated as an electron transfer by carrying out the enzyme reaction in the presence of the substrate, for example. The electron transfer can be measured as a current through applying a voltage to an electrode, for example.

The analytical method according to the present invention may further include a removing step of removing streptavidin which does not bind to the second nucleic acid molecule by washing after adding the sample and the streptavidin, for example.

The analytical method according to the present invention may further include an adding step of adding a substrate. The order of adding the substrate is not particularly limited, and the substrate may be added at the same time as or after adding the sample and the labeled streptavidin or may be added before or after adding the labeled streptavidin after adding the sample, for example. In the case where the analytical device includes the reagent section, it is preferred that the labeled streptavidin is added after adding the sample, for example.

While the invention has been particularly shown and described with reference to exemplary embodiments thereof, the invention is not limited to these embodiments. It will be understood by those of ordinary skill in the art that various changes in form and details may be made therein without departing from the spirit and scope of the present invention as defined by the claims.

This application is based upon and claims the benefit of priority from Japanese patent application No. 2010-287590 filed on December 24, 2010, the disclosure of which is incorporated herein in its entirety by reference.

### Industrial Applicability

According to the present invention, an object to be analyzed can be simply analyzed. An aptamer technology can be applied to the present invention, for example. Aptamers are obtained in a test tube, for example. Therefore, aptamers capable of binding to a highly toxic compound and aptamers capable of binding to a low molecular compound can be obtained. Thus, according to the present invention, problems with a method using an antigen-antibody reaction and the like can be solved, and a simple analysis by signal detection such as electrochemical detection or the like can be performed, for example.

### Explanation of reference numerals

- 1: analytical device
- 10: basal plate
- 20: electrode
- 40: nucleic acid element
- 41: first nucleic acid molecule
- 42: second nucleic acid molecule
- 43: intervening linker
- 44: first additional linker
- 45: second additional linker
- 50: target

## Claims

1. An analytical device comprising:
a basal plate;
a nucleic acid element; and
a detection section of detecting a signal, wherein
the nucleic acid element and the detection section are arranged on the basal plate,
the nucleic acid element comprises a first nucleic acid molecule and a second nucleic acid molecule,
the first nucleic acid molecule is a nucleic acid molecule capable of binding to a target,
the second nucleic acid molecule is a nucleic acid molecule capable of binding to streptavidin,
when the target does not bind to the first nucleic acid molecule, a binding capacity of the second nucleic acid molecule to the streptavidin is inactivated,
when the target binds to the first nucleic acid molecule, a binding capacity of the second nucleic acid molecule to the streptavidin is activated, and
the detection section detects binding between the second nucleic acid molecule and the streptavidin.

2. The analytical device according to claim 1, wherein
the first nucleic acid molecule is a nucleic acid molecule whose structure changes by binding of the target to the first nucleic acid molecule, and
the second nucleic acid molecule is a nucleic acid molecule whose structure changes by the change in structure of the first nucleic acid molecule.

3. The analytical device according to claim 1 or 2, wherein
the first nucleic acid molecule and the second nucleic acid molecule are aptamers.

4. The analytical device according to any one of claims 1 to 3, wherein
the second nucleic acid molecule comprises any of the following polynucleotides (a) to(c):
(a) a polynucleotide composed of a base sequence represented by any of SEQ ID NOs: 1 to 10;
(b) a polynucleotide composed of a base sequence obtained by displacement, deletion, addition, and/or insertion of one or more bases in the base sequence (a) and capable of binding to streptavidin; and
(c) a polynucleotide composed of a base sequence having a identity of 50% or more to the base sequence (a) and capable of binding to streptavidin.

5. The analytical device according to any one of claims 1 to 4, wherein
the nucleic acid element is a single-stranded nucleic acid molecule obtained by joining the first nucleic acid molecule and the second nucleic acid molecule to each other.

6. The analytical device according to any one of claims 1 to 5, wherein
the nucleic acid element further comprises a linker.

7. The analytical device according to any one of claims 1 to 6, wherein
streptavidin is labeled streptavidin obtained by binding a labeling substance to streptavidin, and
the signal is derived from the labeling substance.

8. The analytical device according to claim 7, wherein
the labeling substance emits a signal.

9. The analytical device according to claim 7, wherein
the labeling substance is an enzyme which catalyzes an oxidation-reduction reaction.

10. The analytical device according to claim 9, wherein
the labeling substance is the enzyme which catalyzes an oxidation-reduction reaction, and
the signal is emitted from a substrate by the oxidation-reduction reaction.

11. The analytical device according to claim 10, further comprising a reagent section, wherein
the reagent section contains a substrate for the oxidation-reduction reaction.

12. The analytical device according to any one of claims 7 to 11, wherein
the signal is an electrochemical signal.

13. The analytical device according to any one of claims 1 to 12, wherein
the detection section contains an electrode.

14. The analytical device according to any one of claims 1 to 13, wherein
the nucleic acid element is arranged on the detection section.

15. The analytical device according to any one of claims 1 to 14, comprising: two or more of the nucleic acid elements, wherein
the two or more of the nucleic acid elements each has the first nucleic acid molecule which can bind to a different target.

16. An analytical method for analyzing a target using the analytical device according to any one of claims 1 to 15, the analytical method comprising:
an adding step of adding a sample and streptavidin to the analytical device; and
a detecting step of detecting binding between the second nucleic acid molecule and the streptavidin in the detection section, whereby detecting a target.

17. The analytical method according to claim 16, wherein
the streptavidin is labeled streptavidin obtained by binding a labeling substance to streptavidin.

18. The analytical method according to claim 17, wherein
the labeling substance emits a signal.

19. The analytical method according to claim 18, wherein
the labeling substance emits a signal by an oxidation-reduction reaction.

20. The analytical method according to claim 19, wherein
the labeling substance is an enzyme which catalyzes an oxidation-reduction reaction.

21. The analytical method according to claim 20, wherein
the detecting step is performed in the presence of a substrate for the oxidation-reduction reaction.

22. The analytical method according to any one of claims 18 to 21, wherein
the detection in the detecting step is detection of an electrical signal.
